# EUROPÄISCHE PATENTANMELDUNG

(11) **EP 1 683 743 A1**
(43) Veröffentlichungstag der Anmeldung: **26.07.2006**
(21) Anmeldenummer: 05001078.4
(22) Anmeldetag: 20.01.2005
(51) Int. Cl.: B65F 1/00

(54) **Verfahren zur Herstellung eines Behälters und ein danach hergestellter Behälter**

(71) Anmelder: Flexitank Systems Germany GmbH, 08223 Grünbach (DE)
(72) Erfinder: Kowitz, Torsten, 08236 Ellefeld (DE)
(74) Vertreter: Helge, Reiner

(57) **Zusammenfassung**

Die Erfindung betrifft ein Verfahren zur Herstellung eines Behälters und einen danach hergestellten Behälter für den diagnostischen Probenversand sowie für die Entsorgung von Abfällen aus dem Klinik-, Krankenhaus- und Laborbereich.

Der Behälter besteht aus zwei Werkstoffkomponenten und zwar aus einer Außenwandung (9) aus Karton und einer mit einem Einfüllstutzen (2) versehenen Innenwandung (1) in Kissenform aus einer doppelwandigen Kunststoff-Folie, wobei in den Zwischenraum der Kunststoff-Folien ein flüssigkeitsabsorbierendes Material (7) in Form eines Granulates eingebracht ist.

## Beschreibung

Die Erfindung betrifft ein Verfahren zur Herstellung eines Behälters und einen danach hergestellten Behälter für den diagnostischen Probenversand sowie für die Entsorgung von Abfällen aus dem Klinik-, Krankenhaus- und Laborbereich.

Im Klinik- und/oder Krankenhausbereich fallen im erheblichen Maße immer wieder Flüssigkeiten und/oder Feststoffe bei medizinischen Eingriffen an. Ebenso kommt es im Laborbereich zu einer relativ großen Menge vom Abfällen von Flüssigkeiten und/oder Feststoffe nach Abschluß der Laboruntersuchungen. Diese Abfälle betreffen beispielsweise Blut oder Gewebeteile, sehr oft auch infektiöse Materialien, welche, wenn keinerlei Verwendung mehr vorgesehen ist, entsorgt werden müssen. Hierfür kommen thermische Sondermüll-Entsorgungsverfahren zur Anwendung.

Für das Aufnehmen und kurzzeitige Aufbewahren bis zur Entsorgung bzw. für den Transport werden diese Abfälle in speziell hierfür vorgesehene Behälter gefüllt. In bevorzugter Weise sind diese Behälter aus Glas, wobei diese als Einwegartikel konzipiert sind und im gleichen thermischen Prozeß mit entsorgt werden.

Diese Entsorgungsbehälter aus Glas haben mehrere Nachteile. Einmal besteht ein erhöhtes Risiko der Bruchgefahr; zum einen beim Transport vom Hersteller über den Fachhandel zum Nutzer (Klinik, Krankenhaus, Labor) und zum anderen im befüllten Zustand vom Nutzer zum Entsorger. Oftmals werden diese Behälter deshalb in spezielle Transportbehälter eingesetzt, z.B. in Körbe, die mehr oder weniger der äußeren Form der Entsorgungsbehälter angepaßt sind.

Ein zweiter Nachteil derartiger Entsorgungsbehälter besteht darin, daß diese ein sehr großes Transport- und Lagervolumen benötigen.

Desweiteren muß bei der thermischen Entsorgung dieser Behälter mit einem sehr hohen Temperaturniveau gearbeitet werden, so daß hierfür ein sehr hoher Energieverbrauch zu verzeichnen ist. Ferner bleibt nach der thermischen Entsorgung noch eine erhebliche Menge an Glasschlacke zurück, die ebenfalls entsorgt werden muß.

Aus der DE 42 43 678 A1 ist ein Behälter bekannt, dessen Behälterwandung gegen eine außen um die Behälterwandung angeordnete Spiralfeder im unbefüllten Zustand ziehharmonikaartig zusammengedrückt bzw. zusammengefaltet ist und durch Entriegelung der am Behälterboden vorgesehenen Halteklappen durch die Spiralfeder für das Befüllen aufgerichtet werden kann. Eine derartige Ausführungsform bringt zwar den Vorteil, daß das Transport- und Lagervolumen im unbenutzten Zustand wesentlich verkleinert wird. Als nachteilig muß jedoch die Spiralfeder angesehen werden, die die Herstellungskosten für den Behälter erhöht und nach dem thermischen Entsorgungsprozeß als ausgeglühtes Schrottstück in den Verbrennungsrückständen verbleibt.

Für den diagnostischen Versand von Proben werden einwandige Kunststoff- oder Glasgefäße verwendet, die in spezielle Transportbehälter eingesetzt werden. Diese haben ein relativ großes Transport- und Lagervolumen.

Für die einwandigen Kunststoff- und Glasgefäße besteht ein erhöhtes Risiko der Bruchgefahr. Bei einem Bruch können sich zum einen die verschiedenen Inhaltsstoffe vermischen und zum anderen können die Inhaltsstoffe in die Umgebung gelangen, was bei mit Krankheitserregern belasteten Proben zu großen Risiken für die Umwelt führen kann.

Aus dem Gebrauchsmuster DE 203 03 981 U1 ist ein Behälter für die Entsorgung von Abfällen aus dem Klinik-, Krankenhaus- und Laborbereich bekannt, welcher im Anlieferungszustand in zusammengeklappter Form vorliegt und zum Befüllen aufgeklappt wird, wobei der Behälter aus zwei Werkstoffkomponenten, einer Außenwandung aus Karton und mindestens einer Innenwandung aus Kunststoff besteht, dessen Innen- und Außenwandung durch eine Überwurfmutter am Einfüllstutzen miteinander verbunden sind. An zwei sich gegenüberliegenden Seiten der Außenwandung, die als Klappteile ausgebildet sind, ist je eine Lasche angeordnet, welche im aufgestellten Zustand des Behälters in die in der Außenwandung vorgesehenen Öffnungen eingehakt sind und an den weiteren gegenüberliegenden Seiten der Außenwandung Klappteile vorgesehen sind, die ein stabilisierendes Element aufweisen.

Der Nachteil besteht darin, daß bei Verletzung der Innenwandung die sich im Inneren befindliche Flüssigkeit nach außen treten kann.

Diesen Nachteil beseitigt das Gebrauchsmuster DE 20 2004 008 468 U1. Hier wird die Kunststoffinnenwand von einer Hülle aus textilem Material umgeben. Die aus textilem Material bestehende Hülle weist auf der Außenseite eine gas- und flüssigkeitsdichte Beschichtung und auf der Innenseite eine die Flüssigkeit absorbierende Schicht auf.

Derartige textile Materialien sind relativ teuer in der Herstellung und können nicht durch Verschweißen mit der Kunststoffinnenwand verbunden werden.

Der vorliegenden Erfindung liegt daher die Aufgabe zugrunde, ein Verfahren anzugeben, mit dem es möglich ist, die eingangs genannten Behälter für den diagnostischen Probenversand sowie für die Entsorgung von Abfällen aus dem Klinik-, Krankenhaus- und Laborbereich preisgünstig herzustellen, wobei diese bei der thermischen Entsorgung auf einen relativ niedrigem Temperaturniveau entsorgbar sind. Die Behälter sollen einen hohen Sicherheitsstandard und im ungefüllten Zustand ein geringes Transport- und Lagervolumen aufweisen.

Erfindungsgemäß wird die Aufgabe durch ein Verfahren und einen danach hergestellten Behälter gemäß den Patentansprüchen 1 und 2 gelöst. Weitere vorteilhafte Ausführungen sind den Unteransprüchen zu entnehmen.

Die Vorteile der Erfindung liegen darin, daß einerseits durch die gefaltete Form vor der Benutzung des Behälters eine geringe Transport- und Lagerkapazität benötigt wird und andererseits die Möglichkeit besteht, Außen- und Innenwandung kurz vor der thermischen Entsorgung wieder voneinander zu trennen.

Als einer der wesentlichen Vorteile dieser erfindungsgemäßen Lösung ist die Verwendung von thermisch leicht zu entsorgenden Materialien für die Behälterwandungen, weil durch die möglich gewordene Reduzierung der Verbrennungstemperaturen beim thermischen Entsorgungsprozeß eine wesentliche Einsparung an Energie erzielt werden kann.

In vorteilhafter Weise kommt für die Innenwandung aus Kunststoff Polyethylen zum Einsatz, weil diese Werkstoffgruppe relativ schadstoffarm thermisch entsorgt werden kann und so die Umweltbelastung durch diese thermische Entsorgung gering bleibt.

Eine weitere vorteilhafte Ausgestaltung der Erfindung kann darin bestehen, daß die Innenwandung mehrschichtig mit zwischen den Schichten eingebrachten flüssigkeitsabsorbierenden Material ausgeführt ist, was insbesondere für eine großvolumige Ausführungsform (über ca. 10 Liter) eine größere Sicherheit gegen ungewollte Leckagen bietet.

Anhand eines Ausführungsbeispieles wird die Erfindung näher beschrieben. Es zeigen
Figur 1 - Schnitt durch die Innenwandung
Figur 2 - Draufsicht des geöffneten Behälters

Die Herstellung der kissenförmigen Innenwandung 1 erfolgt in der Weise, daß von je einem Folienrollenpaar die Folienbahnen b1, a1 und a2, b2 abgezogen werden, wobei die Folienbahnen b1, a1 die obere Seite 13 und die Folienbahnen a2, b2 die untere Seite 14 der kissenförmigen Innenwandung 1 bilden. In die obere Seite 13 wird ein Loch gestanzt, in welches von unten der Einfüllstutzen 2 eingeführt und mit den beiden Folienbahnen b1, a1 verschweißt wird. Anschließend werden die aus den Folienbahnen gebildeten oberen 13 und unteren Seiten 14 übereinandergezogen, in Abhängigkeit von der Volumengröße auf Maß geschnitten und an drei Seiten die Randbereiche miteinander verschweißt.

Daran anschließend wird in die Zwischenräume der Folienbahnen b1, a1 und a2, b2 der oberen 13 und unteren Seite 14 ein flüssigkeitsabsorbierendes Material 7 in Form eines Granulates eingefüllt. Die eingefüllte Menge ist so bemessen, daß sie im Falle einer Verletzung der kissenförmigen Innenwandung 1 den gesamten eingefüllten Inhalt aufnehmen kann.

Nach der Befüllung mit dem flüssigkeitsabsorbierenden Material 7 werden die offenen Bereiche der oberen 13 und unteren Seiten 14 der kissenförmigen Innenwandung 1 miteinander verschweißt, so daß sich eine umlaufende Schweißnaht 15 bildet.

In die separat hergestellte Außenwandung 9 des Behälters aus Karton ist eine im Abstand zur Oberkante versetzt angeordnete klappbare Innenlage 12 vorgesehen.

Diese weist eine in etwa bis zur Mitte gehende Aussparung 10 auf. In diese Aussparung 10 wird die kissenförmige Innenwandung 1 eingeschoben, wobei diese mittels eines am Einfüllstutzen 2 angeordneten Doppelflansches 5 gehalten wird.

Zwischen den einzelnen Flanschplatten des Doppelflansches 5 ist ein Vierkantbereich 6 vorgesehen, dessen Abmessung mit der Breite der Aussparung 10 korrespondiert. Dadurch wird ein Verdrehen der kissenförmigen Innenwandung 1 beim Öffnen oder Schließen des Schraubdeckels 3 sicher verhindert.

Der Behälter besteht aus zwei Werkstoffkomponenten.

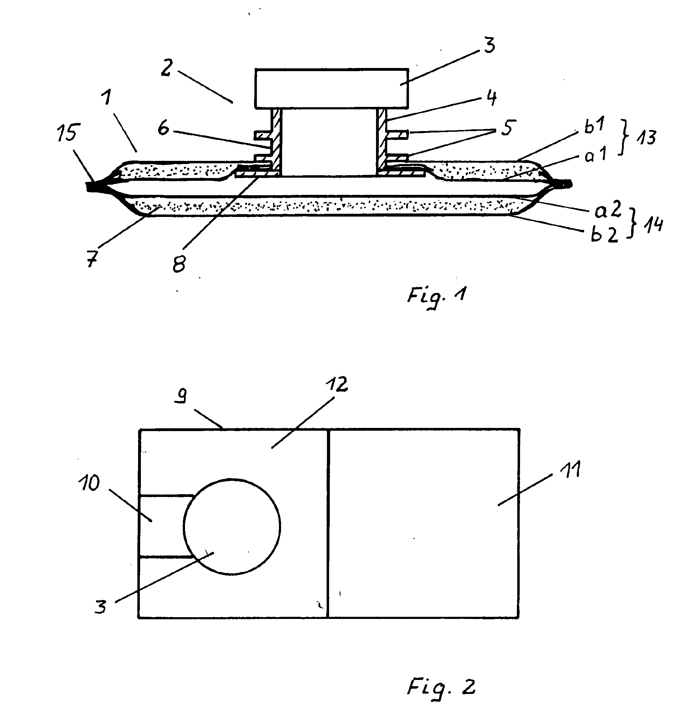

Die Außenwandung 9 des Behälters ist aus Karton hergestellt und liegt im Lieferzustand in zusammengeklappter Form vor. Die Innenwandung 1 in Kissenform ist über den Doppelflansch 5 mit der Innenlage 12 verbunden. Dies wird dadurch erreicht, daß die Innenlage 12 eine Aussparung 10 aufweist, in die die kissenförmige Innenwandung 1 einschiebbar ist - **Figur 2** -.

Die Innenwandung 1 - **Figur 1** - besteht aus einer mehrschichtigen Kunststoff-Folie, wobei zwischen den Folienschichten ein flüssigkeitsabsorbierendes Material 7 in Form eines Granulates eingebracht ist. Die mehrschichtigen Kunststoff-Folien bilden jeweils eine obere 13 und eine untere Seite 14, wobei in die obere Seite 14 der Einfüllstutzen 2 eingebracht ist. Dieser besteht aus dem unteren Flansch 8, einem darüber angeordneten Doppelflansch 5 und einem Gewindebereich 4 mit dem Schraubdeckel 3. Zwischen den einzelnen Flanschplatten des Doppelflansches 5 ist ein Vierkantbereich 6 vorgesehen, der nach dem Einschieben in die Aussparung 10 als Verdrehsicherung fungiert.

Die Außenwandung 9 des Behälters besteht aus einem faltbaren Karton, wobei eine vom oberen Rand nach unten versetzte Innenlage 12 mit der Aussparung 10 vorgesehen ist. Dies dient dazu, den Einfüllstutzen 2 aufzunehmen, so daß dieser vom Deckel 11 im geschlossenen Zustand abgedeckt wird und der Behälter im aufgerichteten Zustand einfach stapelbar ist.

In einer weiteren Ausführung kann der Einfüllstutzen 2 mit einem Gitter oder einer Lochscheibe versehen sein, wobei der Stutzen über den Flansch 8 hinaus verlängert ist und mit einer unteren Scheibe abschließt. Die Verlängerung ist umlaufend mit Durchbrüchen versehen, die mit dem Innenraum der kissenförmigen Innenwandung 1 in Verbindung stehen. Die Gitter oder Lochscheiben nehmen Reagenzröhrchen, Monovetten oder ähnliche schmale Gefäße auf. Der Schraubdeckel des Einfüllstutzens wird dabei mit einem dicht abschließenden Einsatz, der mittig angeordnet ist, aus Gummi oder Kautschuk versehen, so daß dieser mit einer Kanüle durchstoßbar ist und Flüssigkeiten hineingefüllt oder entnommen werden können.

Der abschließende Einsatz im Schraubdeckel verhindert das Austreten von in den Reagenzröhrchen, Monovetten oder ähnlichen eingestellten Flüssigkeiten untereinander.

Die erfindungsgemäßen Behälter können für den Probenversand eine Volumengröße von 100 ml bis 1000 ml und für die Entsorgung von Abfällen Volumengrößen von 2 bis 20 Litern aufweisen. Es sind aber auch andere Volumengrößen denkbar.

Bezugszeichen
- 1 -: Innenwandung in Kissenform
- 2 -: Einfüllstutzen
- 3 -: Schraubdeckel
- 4 -: Gewindebereich
- 5 -: Doppelflansch
- 6 -: Vierkant
- 7 -: Granulat / flüssigkeitsabsorbierendes Material
- 8 -: Flansch
- 9 -: Außenwandung
- 10 -: Aussparung
- 11 -: Deckel
- 12 -: Innenlage
- 13 -: obere Seite
- 14 -: untere Seite
- 15 -: Schweißnaht
- a1 -: Folienbahn
- a2 -: Folienbahn
- b1 -: Folienbahn
- b2 -: Folienbahn

## Patentansprüche

1. Verfahren zur Herstellung eines Behälters für den diagnostischen Probenversand sowie für die Entsorgung von Abfällen aus dem Klinik-, Krankenhaus- und Laborbereich, mit einer faltbaren Außenwandung und einer darin eingesetzten Innenwandung
**dadurch gekennzeichnet, daß**
zur Fertigung der Innenwandung (1) in einem ersten Schritt von jeweils zwei Folienrollenpaaren die Kunststoff-Folien (b1, a1; a2, b2) abgezogen werden, wobei diese eine obere (13) und eine untere Seite (14) der kissenförmigen Innenwandung (1) bilden, in die obere Seite (13) ein Loch eingestanzt wird, in das ein Einfüllstutzen (2) befestigt wird und anschließend obere (13) und untere Seite (14) übereinandergezogen werden, gleichzeitig in Abhängigkeit von der Volumengröße auf Maß geschnitten werden und an drei Seiten die Randbereiche miteinander verschweißt werden, in einem zweiten Schritt die Zwischenräume mit dem flüssigkeitsabsorbierenden Material (7) befüllt und anschließend die offnen Bereiche der oberen (13) und unteren Seite (14) der kissenförmigen Innenwandung (1) miteinander verschweißt werden.

2. Nach dem Verfahren gemäß des Anspruches 1 hergestellter Behälter für die Entsorgung von Abfällen aus dem Klinik-, Krankenhaus- und Laborbereich sowie für den diagnostischen Probenversand, welcher im Anlieferungszustand in zusammengeklappter flacher Form vorliegt und zum Befüllen aufgeklappt wird und dessen Wandungen aus zwei Werkstoffkomponenten, einer Außenwandung aus Karton und mindestens einer Innenwandung aus Kunststoff, versehen mit einem Einfüllstutzen (2), besteht,
**dadurch gekennzeichnet, daß**
die Innenwandung (1) des Behälters in Kissenform ausgebildet ist und dessen obere (13) und untere Seite (14) aus einer mehrschichtigen Folie bestehen, wobei zwischen den Folieschichten ein flüssigkeitsabsorbierendes Material (7) eingebracht ist.

3. Behälter nach Anspruch 2,
**dadurch gekennzeichnet, daß**
das flüssigkeitsabsorbierende Material (7) ein Granulat ist.

4. Behälter nach Anspruch 2 und 3,
**dadurch gekennzeichnet, daß**
in der Oberseite der in Kissenform ausgebildeten Innenwandung (1) ein Einfüllstutzen (2) mit Außengewinde und Schraubdeckel (3) angeordnet ist.

5. Behälter nach Anspruch 2 bis 4,
**dadurch gekennzeichnet, daß**
am Einfüllstutzen (2) ein Doppelflansch (5) angeordnet ist, wobei zwischen den einzelnen Flanschplatten der Stutzen aus Vierkant (6) ausgebildet ist.

6. Behälter nach Anspruch 2 bis 5,
**dadurch gekennzeichnet, daß**
im Einfüllstutzen (2) ein Filter oder eine Lochscheibe angeordnet ist.

7. Behälter nach Anspruch 2 bis 6,
**dadurch gekennzeichnet, daß**
der Schraubdeckel (3) mit einem dicht abschließenden Einsatz aus Gummi oder Kautschuk versehen ist, über welchen Flüssigkeiten mit Hilfe einer Kanüle entnehmbar oder befüllbar sind.

8. Behälter nach Anspruch 2 bis 7,
**dadurch gekennzeichnet, daß**
die Außenwandung (9) aus einem faltbaren Karton besteht, wobei eine vom oberen Rand nach unten versetzte Innenlage (12) mit einer bis etwa zur Mitte reichenden Aussparung (10) angeordnet ist.

9. Behälter nach Anspruch 2 bis 8,
**dadurch gekennzeichnet, daß**
die Breite der Aussparung (10) den Abmessungen des Vierkants (6) entspricht.
